(19) Europäisches Patentamt
European Patent Office
Office européen des brevets

(11) **EP 3 646 791 B1**

(12) **EUROPEAN PATENT SPECIFICATION**

(45) Date of publication and mention
of the grant of the patent:
**29.03.2023 Bulletin 2023/13**

(21) Application number: **18382767.4**

(22) Date of filing: **31.10.2018**

(51) International Patent Classification (IPC):
***A61B 6/00*** *(2006.01)*

(52) Cooperative Patent Classification (CPC):
**A61B 6/542; A61B 6/5294; A61B 6/545**

(54) **METHOD AND AUTOMATION DEVICE FOR THE AUTOMATION OF X-RAY IMAGE ACQUISITION AND VISUALIZATION AT A MINIMUM DOSE**

VERFAHREN UND AUTOMATISIERUNGSVORRICHTUNG ZUR AUTOMATISIERUNG DER RÖNTGENBILDERFASSUNG UND -VISUALISIERUNG BEI MINIMALER DOSIS

PROCÉDÉ ET DISPOSITIF D'AUTOMATISATION POUR L'AUTOMATISATION D'ACQUISITION ET DE VISUALISATION D'IMAGES À RAYONS X À UNE DOSE MINIMALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.05.2020 Bulletin 2020/19**

(73) Proprietor: **Siemens Healthcare GmbH**
**91052 Erlangen (DE)**

(72) Inventor: **Martin Recuero, Ana Belen**
**28231 Las Rozas - Madrid (ES)**

(74) Representative: **Clarke Modet & Co.**
**C/ Suero de Quiñones 34-36**
**28002 Madrid (ES)**

(56) References cited:
**JP-A- 2009 268 801        JP-A- 2017 109 118**
**US-A1- 2016 021 727        US-A1- 2017 209 105**

**Description**

The invention describes a method and an automation device for the automation of X-ray image acquisition and visualization at a minimum dose. The invention also describes a method to create an automation-unit for the automation of X-ray image acquisition and visualization at a minimum dose and such automation-unit.

In the technical field of X-ray image acquisition, it is a goal to achieve the best cost-effective image quality for diagnosis at the lowest dose exposure for the patient, especially to reduce the number of discarded images and reducing the dose applied to the patient. Furthermore, it would be of advantage to automate the X-ray acquisition and processing parameters so that the X-ray systems gain autonomy and at the same time provide with objective values for acquisition and post-processing of X-ray images.

In the state of the art, for optimization of exposure parameters, phantom studies are used, providing a way to test the effect of different parameter settings for dose and image quality, under controlled conditions. For example, management of dose reduction is currently performed with combined applications to reduce exposure or with a knowledge based information retrieval, iteratively incorporating information of the clinical target, system geometry and X-ray statistical information. Other ways to reduce dose take into consideration the LIH (Last Image Hold) for the iterations loops, especially taken into consideration for fluoroscopy. Further applications offer a "Kilovolt assist" by previously doing a scout to adjust the dose based on image quality readjustment or they are based on an organ dose modulation based on X-ray tube current modulation, mainly for superficial tissues, such as breasts. There are also some solutions which use post-patient collimation after analyzing the non-uniform spectral response of X-ray detector. The solutions described are mainly applied to computer tomography ("CT").

It is a disadvantage that this kind of techniques does not account on biological diversity and patient variability.

JP2017007249A discloses radiation dose management techniques for an X-ray imaging apparatus to display, control and update a target dose transmitted through a patient based on deviation of dose indices.

So it is the object of the present invention to improve the known methods and describe an optimized method for the automation of X-ray image acquisition and visualization at a minimum dose.

This object is achieved by the method to create an automation-unit according to claim 1, an automation-unit according to claim 7, an automation-method according to claim 8, an automation device according to claim 12 and a medical X-ray imaging system according to claim 13.

The core of the invention is the automation of X-ray image acquisition and visualization at a minimum dose, especially parameter evaluation for X-ray image acquisition and X-ray image visualization. This is achieved by supervised machine learning applied to automatic X-ray image acquisition and visualization.

To perform the automation of X-ray acquisition and visualization at the minimum dose, a special method is necessary to train an automation-unit that is able for accomplishing this automation.

The method according to the invention to create an automation-unit for the automation of X-ray image acquisition and visualization at a minimum dose comprises the following steps.

- providing a computerized training-unit. This training-unit is designed to train an automation-unit (e.g. an algorithm) with a method of machine learning. It is able to process data for the training, especially to convert data in a suitable form, and for the training itself. The training-unit is e.g. a computer comprising a processor, a memory and a software that is designed to process datasets and train the automation-unit. Preferably, the training-unit comprises a data processor unit designed for the necessary calculations. The data processor unit preferably deals with extracting the training data and processing it as input of the training algorithm in the training-unit.

- providing an automation-unit
  This automation-unit is provided at the training-unit and is generally untrained or at least not optimally trained. Surely, the automation-unit is designed to be trained by the training-unit. The automation-unit could be an algorithm in the memory (e.g. in the Random Access Memory) of the training-unit, but also a programmable hardware device (e.g. a field programmable gate array, FPGA or a programmable Memory, e.g. an EEPROM) brought in data-connection with the training-unit.

- providing X-ray sample datasets
  These X-ray sample datasets are necessary for training and each dataset should comprise all necessary information. However, there surely also could be other datasets not comprising all necessary information or any relevant information at all. These datasets could be ignored or could be used to provide additional statistical information. The datasets could be provided in a memory of the training-unit, a dataset-unit (e.g. a database or a data collection cloud) or a data storage medium. The datasets could be provided via a RIS (Radiology Information System) or a PACS (Picture Archiving and Communication System).

The X-ray sample datasets preferably comprising at least headers of medical image files (e.g. DICOM-headers) and/or other files, wherein DICOM datasets are preferred (DICOM = Digital Imaging and Communications in Medicine). However, There exist other medical image formats and the relevant information has not necessarily be stored in an image file, but in an accompanying file, e.g. a log file or a separate parameter-file. The X-ray sample datasets comprising information (preferably numeric values) about

> a) the Deviation Index or data from which the Deviation Index can be derived and
> b) values of acquisition parameters and/or of processing parameters, and
> c) medical task parameters (e.g. BMI, ROI, displayed object) of a X-ray image file.

It is preferred that the X-ray sample datasets comprise information at least about the Deviation Index or data from which the Deviation Index can be derived and values of acquisition parameters and/or of processing parameters, wherein both, values of acquisition parameters and values of processing parameters, are preferred.

- extract data
  These extracted data comprising the Deviation Index DI and values of predefined acquisition parameters and/or of processing parameters from the X-ray sample datasets. The data are classified in one of the predefined number of data-groups (at least one, preferably more) from the extracted data.

- training of the automation-unit
  The automation-unit is then trained by the training-unit based on the principles of machine learning by using the data-groups.

An automation-unit according to the invention for automating X-ray acquisition and visualization at a minimum dose is manufactured by a method according to the invention as described above.
An automation-method according to the invention for automation of X-ray image acquisition and visualization at a minimum dose, comprises the following steps.

- Providing an automation-unit according to the invention.

- Providing medical task parameters, e.g. the body mass index ("BMI") or the region of interest ("ROI"), about a planned image-acquisition. This could e.g. be achieved by a computation unit able to extract values of acquisition parameters and/or of processing parameters of a medical X-ray imaging system or simply by receiving information about a body part to be examined and the BMI of the patient.

- Calculate optimal values of acquisition parameters and/or of processing parameters based on the training algorithm used and the optimal goal of optimization.

- Providing the calculated optimal values of acquisition parameters and/or of processing parameters to be used for image acquisition.

An automation device according to the invention for automating X-ray acquisition and visualization is designed for using an automation-method according to the invention as described above.
The automation device preferably comprises:

- A data-interface to receive data of an X-ray system.

- A dataset-unit, e.g. a data collection cloud, with datasets comprising headers of medical image files, e.g. DICOM-headers, comprising information about the following numeric values:

> a) the Deviation Index or data from which the Deviation Index can be derived and
> b) values of acquisition parameters and/or of processing parameters,
> c) medical task parameters.

- An automation-unit according to the invention.

- Preferably a training-unit designed to further train the automation-unit.

- A data interface to provide values of acquisition parameters and/or of processing parameters and DI or similar unit for an X-ray system and/or to control an X-ray system.

A medical X-ray imaging system according to the invention comprises a number of X-ray imaging systems and an automation device according to the invention.

For the invention, it is very advantageous to find the optimal values of parameters in a radiography study case which are used to acquire and finally visualize the image by a physician. These are the values of acquisition parameters and/or processing parameters mentioned above. The optimal values of parameters are those which achieve the best image quality at the minimum dose applied. They can be found during the training, e.g. by monitoring weight coefficients chosen to estimate the influence of certain parameters as it is explained in the following. These parameters with the smallest weight coefficients will probably have the least influence on the calculation of the Deviation Index and may, thus, also be ignored in later calculations.

A way, how the parameters can be chosen is explained in the following, beginning with the Deviation Index itself.

The Deviation Index ("DI") measures the variation of the actual Exposure Index ("EI") compared with an ideal Target Exposure Index ("TEI") for a patient and examination. Ideally this Deviation Index should be 0 (or deviate 0% from 0) to follow the ALARA principles ("As Low As Reasonably Achievable"). The Exposure Index EI is the measure of the amount of exposure received by the image receptor ("IR") and is also indicative of the image quality. The way to achieve the idea of the invention is to use supervised machine learning algorithms to find the better combination of parameters towards a Deviation Index DI=0, i.e. the best image quality at the lowest dose possible.

The Deviation Index DI is defined based on the Exposure Index EI and the Target Exposure Index TEI as

$$DI = 10\log\frac{EI}{TEI} \;. \qquad\qquad (1)$$

Due to the logarithmic connection between DI, EI and TEI, data from which the Deviation Index can be derived could comprise EI and TEI or the Exposure factor EI/TEI, since the Deviation Index could easily be derived from these values.

It should be noted that under data from which the Deviation Index can be derived also the percentage Deviation Index $DI(\%) = (EI/TEI-1)\cdot100$ could be understood. It is very advantageous that the Deviation Indices of the different datasets all have the same format (e.g. either DI or DI(%) should be used). In many applications, the Deviation Indices of the different datasets must all have the same format. The "DI" is the output of the formula (1), the "DI(%)" is how much in percentage the actual EI is exceeded or fallen behind from the ideal EI (i.e. the TEI-value). Any of them can be used indifferently (also within the invention), since there is a direct mathematic correlation. Each one is a different way of expressing the same. In the clinical praxis, the DI is more spread out, for the general public DI(%) might be better understood. A value "+3" of the DI indicates an overexposure, meaning double of the ideal exposure index (100%). The most important for any training or data collection is the consistency in the unit.

It must be noted that EI and TEI and even DI may vary if datasets from different manufacturers are used. However, there is a standard for rendering the Exposure Index for digital X-ray imaging systems. Developed concurrently by the International Electrotechnical Commission (IEC) and the American Association of Physicists in Medicine (AAPM), in cooperation with digital radiography system manufacturers, the index has been implemented as an international standard. It is known as the IEC exposure index. The IEC exposure index is unique to the receptor type being used and to the exam performed.

To be considered within the invention, the use of datasets of imaging systems of one single manufacturer is preferred. It is advantageous not to mix up datasets from different manufactures since the EI or TEI may be calculated there in a different way and may depend on the technology underlying. In order to mix datasets from machines from different manufacturers, all datasets are preferably homogenized to provide systematically comparable values parameters EI and TEI and their respective values.

The Deviation Index is generally calculated in radiology and included in the DICOM-header (universal). This factor is very important in radiology for physicians to be aware of exposure indexes, miscalibrations of the equipment, errors in the acquisition, etc. EI and TEI are the parameters necessary to calculate the DI.

According to the invention, it is preferred to calculate a Deviation Index (not the DI given in a dataset and not based on the EI and TEI) on parameters that are the acquisition parameters and processing parameters of this dataset. An automation-unit trained on this calculated Deviation Index is able to provide the optimal values of parameters for an optimal calculated Deviation Index for later image acquisitions: The optimal combination of weighted parameters leads to achieve the goal of DI=0. The combination of the values of parameters can be logarithmic, linear, etc. depending on the machine learning algorithm used, as well as the optimization method to be used to calculate the weights to achieve the optimization goal. By means of machine learning algorithms it is possible to achieve automatic optimal values of parameters to follow the ALARA principle. The optimization goal is a (calculated) DI=0 which measures the variation of the actual EI from the ideal EI (i.e. TEI) to follow this ALARA principle.

The initial DI is usually not zero in a real acquisition (but it could be; DI is normally between -3 and +3). The goal is to achieve a DI = 0 according to ALARA principles. That is why we train the parameter to achieve the best combination to achieve best dose at maximum quality.

With a set of N training examples for a defined study case or patient examination (e.g. body part, Body Mass Index) a model is built up. Given the acquisition parameters AP (number of i parameters) and the processing parameters PP (number of j parameters), and the weight coefficients w (number of i+j parameters) related to a defined study case, a (calculated) percentage Deviation Index $DI(\%)_C$ could be calculated with the formula

$$DI(\%)_C = w_1 AP_1 + ... + w_i AP_i + w_{i+1} PP_1 + ... + w_{i+j} PP_j \qquad (2)$$

Each of the parameters is weighted with a coefficient w in the model. The supervised machine training algorithm aims at iteratively making predictions on the optimal combination of the training data so that the output obtained is a DI(%) of 0%. Learning stops when the algorithm achieves an acceptable level of performance. Since there exist many different supervised training algorithms, and since the selection of the algorithm may influence in the final output of the parameter optimization complexity and thus the number of samples needed, it is preferred to choose the optimal algorithm. It is also preferred to choose a group of different algorithms since different training algorithms may provide different useful information. For example if the algorithm provides power to discriminate between the most relevant features causing a larger DI, the weights of each parameter determine the importance to achieve a DI of 0, the algorithm can also show the relation between the features studied. In this case, the results can be very useful for training personnel in radiological principles, or determine where the system has room for improvement.

As said above, the overall goal of formula (2) regarding the training is to get a (calculated) $DI(\%)_C$ that has a very low value, wherein zero would be ideal.

As an alternative goal to train the automation-unit it is preferred to compare the calculated Deviation Index $DI(\%)_C$ with a comparable value (i.e. e.g. with DI(%)) given in or calculated from the dataset. It is also possible to calculate a value referring to DI from $DI(\%)_C$ by using the equation $10\log([DI(\%)_C/100]+1)$ with equations (1) and (2) and compare this with the DI given in or calculated from the dataset.

It should be noted that the training is not based on the calculation of a DI, but on the estimation of those weight coefficients that could model the correct DI from the acquisition parameters PA and/or processing parameters PP. The goal is to model the DI of a dataset by using the values of acquisition parameters PA and/or processing parameters PP. For reaching this goal, the weight parameters are adjusted until this goal is reached for many datasets with the same set of weight parameters (in one data-group).

By the way, could datasets with a low DI-value give additionally hints how optimal parameters should be chosen for an ideal image-taking.

The equation (2) is a preferred way to represent the supervised machine learning algorithm: the combination of weighted parameters (or a feature vector) to be optimized towards an ideal goal. It would be a preferred goal, to reach a (calculated) $DI_C$=0 from the ideal target exposure which produces the best image at the minimum dose. The parameters AP and PP should be any relevant parameters for a defined study case (acquisition or image processing parameters included), the expression "+...+" is the algebra way to represent a number of parameters without writing them all. Thus, the number of used parameters is at first hand not fixed.

For every study case group, (e.g. body part) a training should be performed with a predefined number of relevant parameters. In summary, the representation/nomenclature of the problem is generalized to adhere to any machine learning algorithm and the X-ray imaging protocol. It could be said that for the calculation of the (calculated) Deviation Index (for a medical task group), a combination of weighted parameters or a "feature vector" is preferably used.

Regarding which acquisition and processing parameters should be used for the training, these must be pre-defined for each study case, each protocol uses a number of parameters (as a general rule mAs (milliamperesecond) and kV (kilovolts) are always present, in many cases windowing). If any doubt, all parameters present in the DICOM header can be used, especially if they change from one DICOM header to another for the same study case; which may happen is that a certain parameter (e.g. filter type is always 0.0, because in the clinical praxis is widely accepted not to be used for a certain examination type).

DICOM headers are preferred datasets. Thus, the X-ray sample datasets preferably comprise DICOM headers. The parameters in the DICOM header are the ones usually used in the acquisition and processing of a medical image. All the information needed is usually included in the DICOM headers, from acquisition to image processing or Deviation Index.

A vast number of X-ray sample datasets (especially of the same format) is preferred for the training of machine algorithms. The expression "vast" means that the number of datasets used for training needs to be representative of the real-world use of the study case, i.e. the clinical praxis. The more datasets are used for training the better the result will be. The selection of the algorithm may influence on the final output of the parameter optimization complexity and thus the number of X-ray sample datasets needed, as well as the number of study parameters (acquisition and processing parameters) used influence the number of sample data needed, so that the output results are reliable.

Regarding the format, it is very advantageous if in the training-unit the Deviation Index (logarithmic Exposure factor) is used, then all the data collected should use DI (and not DI(%) or the Exposure factor). If datasets comprise another format of DI, this value should be converted by a mathematical correlation into the same format the automation-unit is (or was) trained. The same should be performed with all other parameters: e.g. if for the parameter current applied the unit mAs is used during training, all the datasets should be expressed in the same format. In the case a dataset provides the parameter current applied in the format pAs, this should be brought in the format mAs. Thus, it is particularly preferred that the format of the extracted data is always consistent along the entire training and the entire use of the trained automation-unit. Also the number of parameters used should be always the same for the same training case/clinical case. Those parameters to be included in the training/clinical case are preferably defined previous to the training. If any doubt all parameters used in the acquisition/visualization of the clinical case and contained in the header can be used.

It is preferred that all the relevant parameters for a clinical case are included in the training formula (2) so that they can be optimized and achieve integral reliable results. As already said above, parameters that are irrelevant for the computation of the Deviation index can later be identified and excluded by the weight coefficients (that will have a very low value), if this should appear to be necessary. Once a model is defined (by model, the parameters that are chosen from the dataset and the chosen machine learning algorithm are meant), the optimal weighting values are calculated. That is, first the model is built, then the optimal weights are calculated.

In summary, it could be said that during the training the (calculated) Deviation Index of one dataset is calculated following formula (2) such that the goal DI=0 is achieved. This will be done with a vast number of datasets (e.g. parallel or iteratively). One can say that the training is done, when the correct weight factors are found. In praxis, this goal could be reached, when the calculated DI differs from the given DI only by a small fraction (what can be quantified by a threshold value) for the difference or the ratio of the two values, in general terms according to the optimization procedure used in the machine learning procedure. In addition, the trained automation-unit may be able to provide optimal values of parameters learned during training, e.g. those values where the given DI was minimal.

Usually, the automation-unit comprises or is an algorithm that is designed for machine learning. For example is a neuronal network one of the possible machine learning algorithms. However, there are many preferred supervised machine learning algorithms. Depending on the definition of the case one or another is more convenient. Other examples for preferred algorithms are Support Vector Machine or Decision Trees. The automation unit may comprise two or more different algorithms wherein during training the best algorithm is chosen.

The units or devices of the invention mentioned above can be completely or partially realized as software modules running on a processor of a computing system. A realization largely in the form of software modules can have the advantage that applications already installed on an existing system can be updated, with relatively little effort, to install and run the methods of the present application. The object of the invention is also achieved by a computer program product with a computer program that is directly loadable into the memory of a computing system and which comprises program units to perform the steps of the inventive method when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

According to a preferred method, a training model (i.e. an automation-unit) is built by the following steps:

- Combining values of processed predefined acquisition parameters and/or of processing parameters of a X-ray sample dataset that are weighted with weight coefficients. This should be done in such a way that the principles of the provided machine learning algorithm at the training automation-unit are fulfilled.

- Performing a training step with respect to the actual Deviation unit DI of the X-ray sample dataset or a comparable unit value of the X-ray sample dataset which has the same/similar definition.

- Repeating these steps with different X-ray sample datasets.

Given a group of datasets (observations or training dataset), the automation-unit should learn how to optimally combine the information from the different predefined acquisition parameters and processing parameters to achieve the best clinical image quality at the minim dose. This should be done following the ALARA principles (As Low As Reasonably Achievable) for a medical task. To this end, the DI should ideally be "0" or in praxis at least as low as possible to achieve the optimization goal according to the clinics.

According to a preferred method, a Deviation Index is calculated based on the acquisition parameters and/or the processing parameters (according to above formula (2), wherein the calculated Deviation Index represents a value that is comparable to the Deviation Index DI according to formula (1) or the Exposure factor EI/TEI or DI(%). The values of the predefined acquisition parameters and/or processing parameters of one dataset are combined by using weight coefficients $w_k$.

According to a preferred method, for the training of the automation-unit datasets referring to essentially identical medical task parameters, e.g. same body part, same range body mass index BMI, are grouped in a data-group. In a data-group, preferably in every data-group, the same combination of acquisition parameters and/or processing parameters over the datasets of this data-group is applied for training. It is preferred that the weight coefficients are calculated by the learning of the training of the algorithm (i.e. the Automation-unit) .

According to a preferred method, from the X-ray sample datasets the input data for the training-unit and the information to form dataset groups is extracted.

This should be done with one or more of the following steps:

- The acquisition parameters comprise parameters used during an acquisition of a X-ray image sample, preferably parameters chosen from the group comprising collimation parameters, mAs-parameters, ExposureCorrection-parameters and filter-parameters.

- The processing parameters comprise parameters which can be used to process an image sample, preferably parameters chosen from the group comprising Amplification-parameters, Windowing-parameters, Harmonization-parameters and Gradation-parameters.

- An X-ray parameter which has an influence over the dose or image quality is preferably introduced into the training-unit with the premise of repeating all steps in the training and automation-unit.

- The actual DI is calculated and stored by the medical imaging system for each X-ray sample dataset or in turn the actual DI can be derived from data provided from the medical imaging system when an acquisition has been performed. As said above, the actual DI or comparable unit preferably has data consistency all over the automation procedure.

Dataset group information can commonly be found within the patient registration application in imaging systems and/or stored in the header data (e.g. DICOM Header). Any other medical task parameter may be derived from the previous data and/or from the learning output of the training algorithm. All confidential data should be treated accordingly.

According to a preferred method, the X-ray sample datasets are preprocessed according to the principles of data preparation for machine learning, wherein the extracted data is preferably homogenized (if necessary), e.g. wherein physical units are adjusted, and/or wherein a part of the data is especially converted in a predefined form, according to the machine learning method to be used and/or arranged in a feature-vector. The feature-vector is preferably normalized.

According to a preferred method, to search for the best machine learning method for the invention, at least two different algorithms for machine learning are used during a test-training phase, wherein the results of the algorithms are compared with each other based on machine learning principles with respect to the performance and complexity metrics (by accuracy of modelling the given DI) and the algorithm with the best metric results is chosen for further training of the automation-unit.

According to a preferred automation-method, a value of an acquisition parameter and/or of a processing parameter is manually adjusted by a user, followed by the steps:

- calculation of a Deviation Index DI by the automation-unit,
- comparing the calculated Deviation Index DI with an acceptable range for the Deviation Index DI,
- provide a correction or a warning if the calculated Deviation Index DI exceeds the acceptable range.

According to a preferred automation-method, an image acquisition is automatically started with the adjusted and/or the calculated optimal values of acquisition parameters and/or of processing parameters if the patient is positioned correctly.

According to a preferred automation-method, comprises the steps:

- providing an interface to the automation-unit,
- providing the calculated optimal values of acquisition parameters and/or of processing parameters preferably stored in a data base in a service cloud as the results of the training-unit for different medical tasks and to be further used for the automatic image acquisition and visualization,
- providing a scheduler to update the data provided to the automation-unit at the customer site.

Wherever not already described explicitly, individual embodiments, or their individual aspects and features, can be combined or exchanged with one another without limiting or widening the scope of the described invention, whenever such a combination or exchange is meaningful and in the sense of this invention. Especially some features described here could form individual inventions, especially in combination with other features of this description. Advantages which are described with respect to one embodiment of the present invention are, wherever applicable, also advantageous of other embodiments of the present invention.

Other objects and features of the present invention will become apparent from the following detailed descriptions considered in conjunction with the accompanying drawings. It is to be understood, however, that the drawings are designed solely for the purposes of illustration and not as a definition of the limits of the invention.

Fig. 1 is a schematic illustration of an imaging system with an embodiment of the inventive control equipment in order to perform the method.

Fig. 2 is a flowchart for an embodiment of the inventive method.

Fig. 3 is a flowchart for an embodiment of the inventive automation-method.

Fig. 4 is a flowchart for a cloud implementation of the inventive method.

Fig. 5 shows an example for data preparation.

Fig. 6 shows a flowchart for optimal parameter calculation.

Fig. 7 is a flowchart for an embodiment of the inventive automation-method.

Fig. 8 shows tables with exemplary values.

In the diagrams, like numbers refer to like objects throughout. Objects in the diagrams are not necessarily drawn to scale.

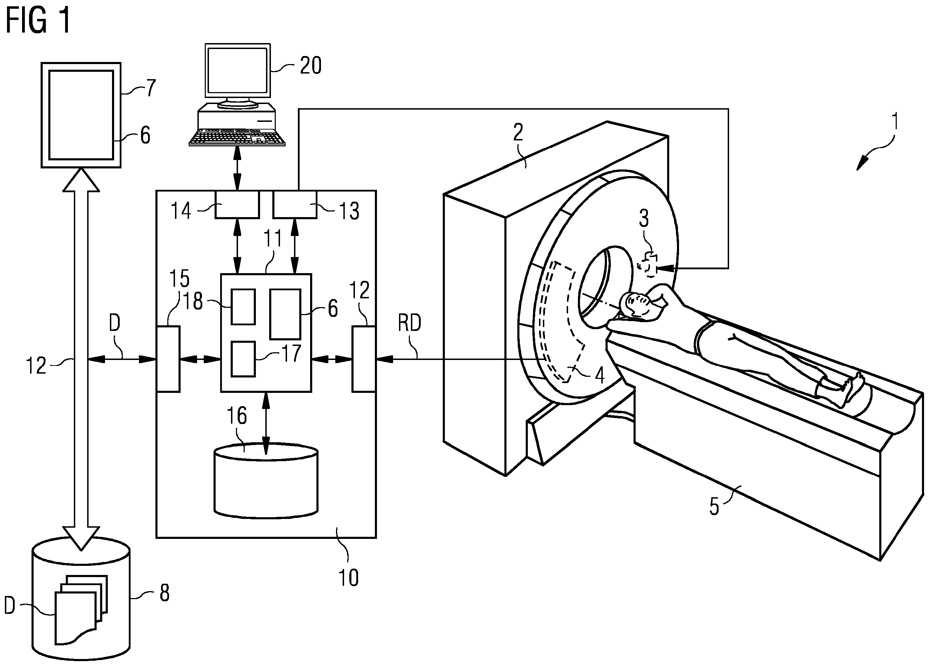

Figure 1 shows a rough diagram of a CT scan system 1 having control equipment 10 to perform the invention-based method. In customary fashion, the CT scan system 1 features a scanner 2 having a gantry in which an X-ray source 3 is rotating which radiates a respective patient who is moved on a stretcher 5 into the measuring room of the gantry so that the radiation hits a detector positioned opposite of the respective X-ray source 3. Special emphasis is placed on the fact that the embodiment according to this figure is merely an example of a CT scanner. It is also possible to use the invention on any CT construction having, for example, circular fixed X-ray detectors and/or several X-ray sources.

In the same way, in the control equipment 10, only those components are represented which contribute to explaining the invention. Basically, such CT systems and respective control equipment are known to the expert and must therefore not be explained in detail.

In this context, a basic component of the control equipment 10 is a processor 11 on which different components are realized in the form of software modules. The control equipment 10 also has a terminal interface 14 to which a terminal 20 is connected by means of which a user can operate the control equipment 10 and, consequently, the CT scan system. A further interface 15 is the network interface to connect to a data bus 21 in order to establish a connection to a RIS (Radiology Information System) or PACS (Picture Archiving and Communication System) and here also to a training-unit 7.

By means of this data bus 21, the datasets D for training that are stored for example in an additional database 8 could be provided to the training-unit 7. In addition, medical task parameters about a planned image acquisition could be provided for the trained automation-unit 6 that is still in or at the training-unit 7 (e.g. for further training). Alternatively, the trained automation-unit 6 could be included in the processor 11. Both alternatives are shown in this figure.

Via a control interface 13, the scanner 2 can be actuated by the control equipment 10, i.e., controlling, for example, the rotation speed of the gantry, the adjustment of the patient's stretcher 5 and even the X-ray source 3. Via an acquisition interface 12, the raw data RD are read from the detector 4. The control equipment 10 has also a storage unit 16 which could store different measuring protocols.

Among other things, a measuring control unit 17 has been implemented as a software component on the processor 11. On the basis of one or several selected measuring protocols, which, if required, have been modified by the user via the terminal 20, this measuring control unit 17 actuates by means of the control interface 13 the scanner 2 in order to perform a measurement and to acquire data.

A further component on the processor 11 is an image data reconstruction unit 18 by means of which the required image data is being reconstructed via the raw data RD obtained from the data acquisition interface 12.

As already said above, the training of the automation-unit 6 could completely be performed with a communication between the database 8 and the training-unit 7 via the data bus 21. After the training, the trained automation-unit could remain in the training-unit 7 that could also arrange the format of the later used parameters accordingly or to further train the automation-unit 6. In this case, the CT scan system 1 could communicate with the automation-unit 6 over the training-unit 7 via the data bus 21 and send medical task parameters to the automation-unit for the automation method. Alternatively, after training the automation-unit 6 could be installed in the processor 11 of the CT scan system 1 and directly receive medical task parameters there.

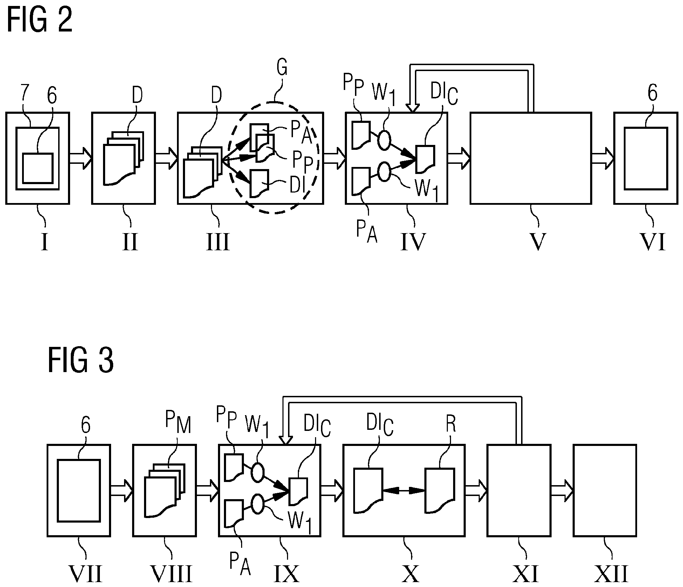

Figure 2 shows a preferred example for a method to create an automation-unit 6 for the automation of X-ray image acquisition and visualization at a minimum dose.

In step I, providing a computerized training-unit 7 is provided together with an (untrained) automation-unit 6. The training-unit 7 is designed to train the automation-unit 6 with a method of machine learning.

In step II, a number of X-ray sample datasets D is provided comprising information about the Deviation Index DI and values of acquisition parameters $P_A$ as well as values of processing parameters $P_P$ of an X-ray image file.

In step III, data from the X-ray datasets D is extracted, wherein the extracted data comprises the Deviation Index DI, values of predefined acquisition parameters and processing parameters from the X-ray sample datasets D. In this example, all data from the datasets is derived from similar medical examinations and, thus, the extracted data can be included in one single data-group G. If there would be datasets from different medical examinations, more than one data-group G should be formed, wherein data from similar examinations should be grouped in the same data-group G.

In step IV, values of processed predefined acquisition parameters $P_A$ and of processing parameters $P_P$ of a X-ray sample dataset D are combined with weight coefficients $W_1$, $W_2$ and a calculated Deviation Index $DI_C$ is formed from the weighted values from the acquisition parameters $P_A$ and processing parameters $P_P$.

In step V, the calculated Deviation Index $DI_C$ is compared with the goal. If the calculated Deviation Index $DI_C$ has reached the goal (at least within a predefined range), the training step follows. If the calculated Deviation Index $DI_C$ is not accurate enough, the calculation of step IV is repeated with other weight coefficients $W_1$, $W_2$.

In step VI, the automation-unit 6 is trained with the training-unit 7 based on the principles of machine learning by using the weight coefficients $W_1$, $W_2$.

Figure 3 shows a preferred example for an automation-method for automation of X-ray image acquisition and visualization at a minimum dose.

In step VII, a trained automation-unit 6 is provided (e.g. the one trained in Figure 2).

In step VIII, medical task parameters $P_M$ about a planned image-acquisition are provided. These medical task parameters $P_M$ comprise values of acquisition parameters $P_A$ and processing parameters $P_P$.

In step IX, a calculated Deviation Index $DI_C$ is created by the automation-unit 6. This automation-unit 6 "knows" the best weight coefficients $W_1$, $W_2$ to calculate Deviation Index $DI_C$ from the values of acquisition parameters $P_A$ and processing parameters $P_P$. The goal is to obtain the best cost effective image quality (DI=0).

In step X, the calculated Deviation Index $DI_C$ is compared with a predefined range R, indicating the value the calculated Deviation Index $DI_C$ is allowed to deviate from a certain optimal value, usually zero.

In step XI it is tested, if the calculated Deviation Index $DI_C$ is outside the predefined range R. If the answer is 'yes', then a warning could be sent and the method goes back to step IX with ameliorated values of acquisition parameters $P_A$ and processing parameters $P_P$, testing whether these values result in a better calculated Deviation Index $DI_C$.

In step XII, the calculated optimal values for acquisition parameters $P_A$ and processing parameters $P_P$ are provided to be used for image acquisition.

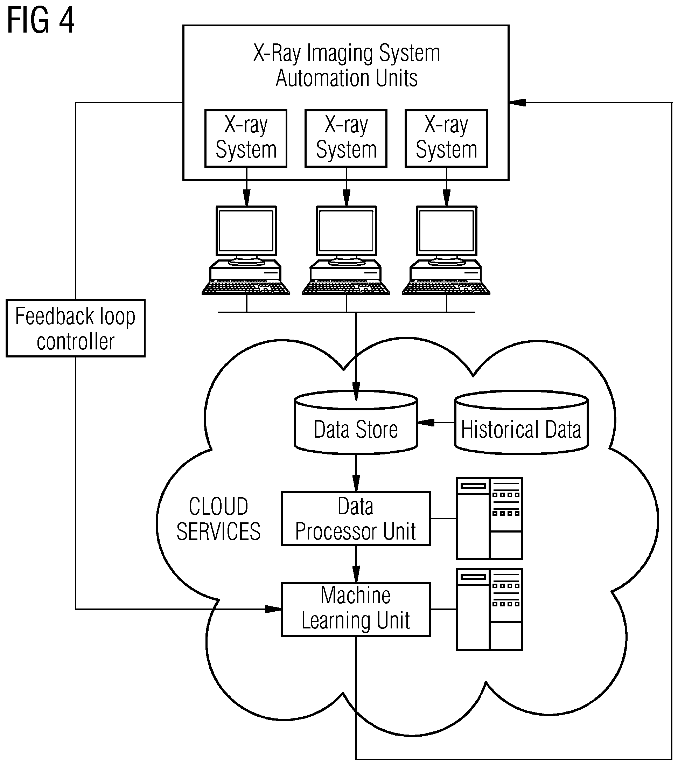

Fig. 4 is a flowchart for a cloud implementation of the inventive method. Data from X-Ray Systems is stored in a Datastore being part of a cloud service. This Cloud Service could also comprise Historical Data that could be added to the data stored. The data stored belong to the X-ray sample datasets D described above.

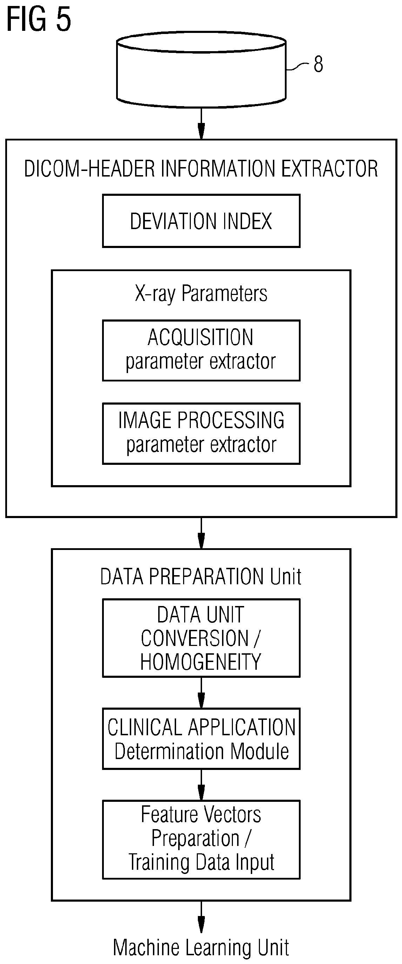

A Data Processor Unit extracts and prepares the data (see Figure 5) and a Machine Learning Unit trains the automation-unit 6 (not shown here). Both, Data Processor Unit and Machine Learning Unit could be part of the training-unit 7. A Feedback Loop Controller could give a feedback to the controllers of the X-Ray Systems based on the processed data.

Fig. 5 shows an example for data preparation. DICOM Data from a database 8 is processed by a DICOM Header Information Extractor, wherein the Deviation Index is extracted and further X-Ray Parameters, acquisition parameters and image processing parameters are extracted (here with a special acquisition parameter extractor and image processing parameter extractor).

After that, the extracted values are processed by a Data Preparation Unit, wherein a Data Unit makes a conversion to the correct format and establishes a homogeneity of the extracted data. Then, the data is processed by a clinical Application Determination Module, checking the clinical application, and a feature vector is created afterwards. Now the extracted data is ready to be used for training of the automation-unit 6 and is given to a Machine Learning Unit via a Training Data Input.

This Extraction and preparation of data may be performed outside the training-unit (then the training-unit 7 would comprise only the Machine Learning Unit), however it is preferred that the training-unit 7 is also able to perform these steps for data extraction and preparation.

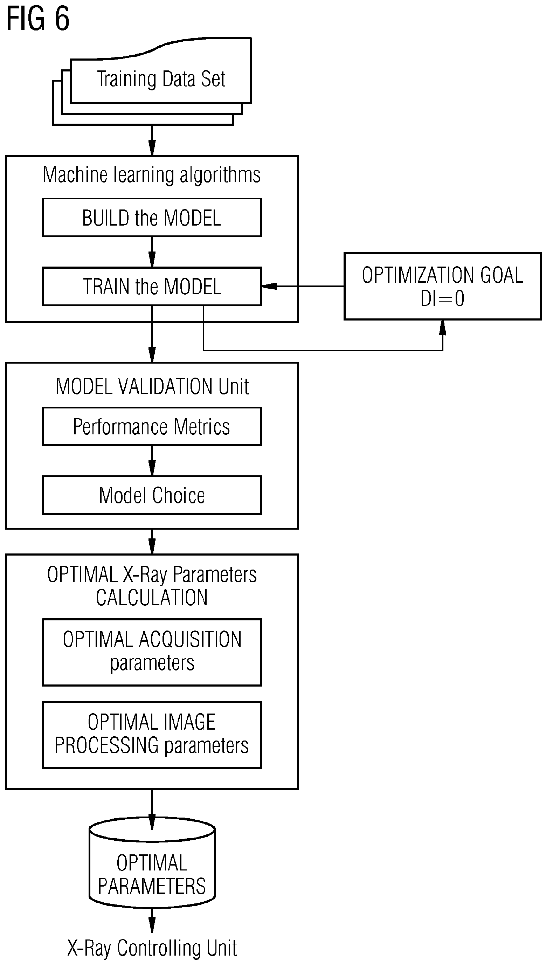

Fig. 6 shows a flowchart for optimal parameter calculation. First, there is a training dataset (that belongs to the X-ray sample datasets D) fed into the process. Machine Learning algorithms are trained on the training dataset by building a model and training this model, wherein the optimization goal is a DI of 0.

After that, the results are given to a Model Validation Unit, where a Performance Metrics and a Model choice is performed. The best Model is chosen here as Machine Learning algorithm chosen for the automation-unit 6.

After that, an optimal X-Ray Parameters Calculation is performed, wherein optimal values of acquisition parameters $P_A$ and processing parameters $P_P$ are calculated. These optimal parameter values are stored as Optimal Parameters and given to the X-Ray Control Units

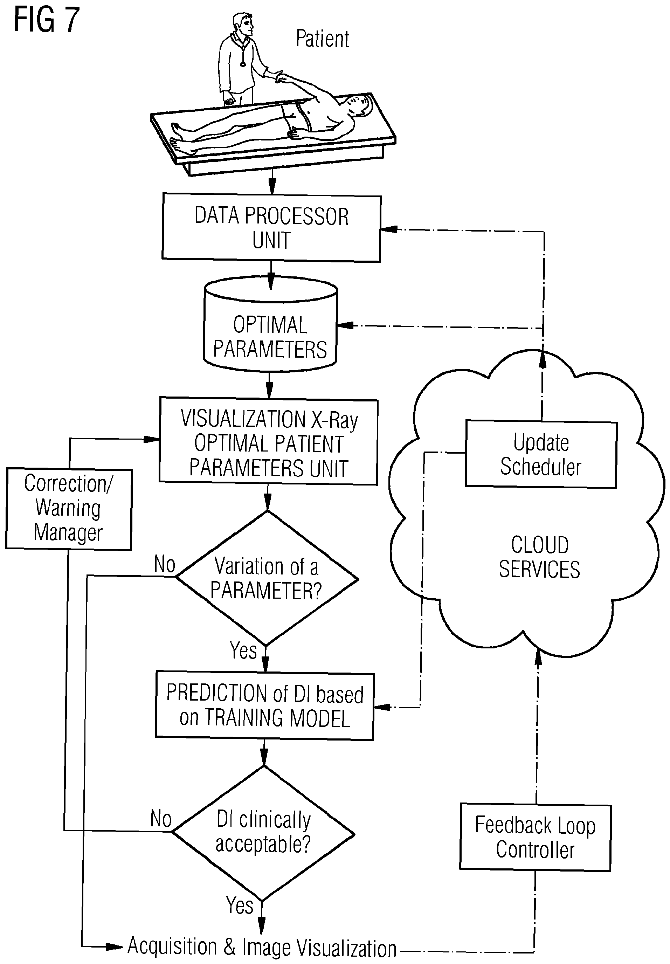

Fig. 7 is a flowchart for an embodiment of the inventive automation-method. In the first step above, data of a patient are prepared as shown in figure 5 by the Data Processor Unit. After that, optimal parameters are calculated as shown and explained in figure 6. These optimal parameters are visualized in a Visualization X-Ray Optimal Patient Parameters Unit. After that, it is decided whether there occurred a variation of a parameter.

If not, the process jumps to an Acquisition and Image Visualization step explained later. If there occurred a variation of a parameter, there is performed a prediction of DI based on the trained automation-unit 6 (a training model). This automation-unit 6 could be updated by an update scheduler, i.e. further trained by a training-unit 7. After the prediction of DI, it is decided whether the predicted DI is clinically acceptable or not.

If not, the process gives a warning controlled by a Correction/Warning Manager and jumps back to the Visualization X-Ray Optimal Patient Parameters Unit. If the DI is clinically acceptable, the method proceeds with the Acquisition and Image Visualization step, where an image is taken and visualized.

The data of this image could be fed back through a Feedback Loop Controller to a cloud service. An Update Controller that is a service in the cloud could add additional aid for the Data Processor Unit and the calculation of optimal parameters.

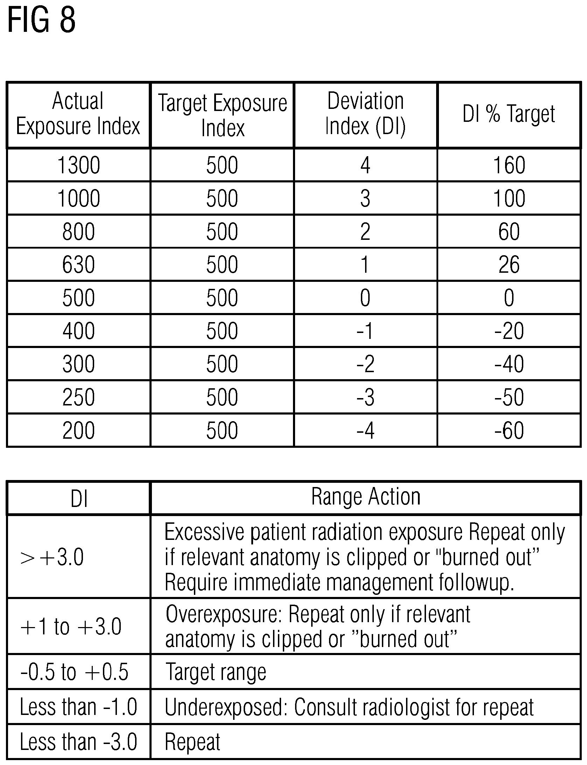

Fig. 8 shows tables with exemplary values. In the upper table, values for the Exposure Index EI are listed in the first column (left), values for the Target Exposure Index TEI are listed in the second column and values for the Deviation Index DI are listed in the third column. The fourth column (right) list the values for the percentage Deviation Index DI%.

In the lower table, there are actions listed ("range actions") that should be performed, if the Deviation Index DI lies in certain value-ranges.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements.

## Claims

**1.** Method to create an automation-unit (6) for the automation of X-ray image acquisition and visualization at a minimum dose comprising the steps:

- providing a computerized training-unit (7), wherein the training-unit is designed to train an automation-unit (6) with a method of machine learning,
- providing an automation-unit (6) at the training-unit (7),
- providing X-ray sample datasets (D) comprising information about

a) the Deviation Index (DI) or data from which the Deviation Index (DI) can be derived,
b) values of acquisition parameters ($P_A$) and/or of processing parameters ($P_P$) and
c) medical task parameters,
of an X-ray image file,

- extracting data from the X-ray datasets (D), wherein the extracted data comprises the Deviation Index (DI) and predefined values of acquisition parameters ($P_A$) and/or of processing parameters ($P_P$) from the X-ray sample datasets,
- forming a number of data-groups (G) from the extracted data, wherein for the training of the automation-unit (6) datasets (D) referring to essentially identical medical task parameters are grouped in a data-group (G);
- training of the automation-unit (6) with the training-unit (7) based on the principles of machine learning by using the number of data-groups (G);

wherein the Deviation Index (DI) measures the variation of the actual Exposure Index (EI) compared with an ideal Target Exposure Index (TEI) for a patient.

2. Method of claim 1, wherein a training model is built by the steps:

- combining values of processed predefined acquisition parameters ($P_A$) and/or processing parameters ($P_P$) of a X-ray sample dataset (D) that are weighted with weight coefficients ($W_1$, $W_2$),
- performing a training step with the combined values with respect to the actual Deviation unit (DI) of the X-ray sample dataset (D) or a comparable unit value of the X-ray sample dataset (D) which has the same/similar definition,
- repeating these steps with different X-ray sample datasets (D).

3. Method as claimed by one of the preceding claims, wherein in a data-group (G) the same combination of values of acquisition parameters ($P_A$) and/or processing parameters ($P_P$) over the datasets of this data-group is applied for training, wherein preferably the weight coefficients ($W_1$, $W_2$) are calculated by the learning of the training of the automation-unit (6).

4. Method as claimed by one of the preceding claims, wherein from the X-ray sample datasets (D) the input data for the training-unit (7) and the information to form data-groups (G) is extracted, wherein:

- the acquisition parameters comprise parameters used during an acquisition of a X-ray image sample, preferably parameters chosen from the group comprising collimation parameters, mAs-parameters, ExposureCorrection-parameters and filter-parameters, and/or
- the processing parameters comprise parameters which can be used to process an image sample, preferably parameters chosen from the group comprising Amplification-parameters, Windowing-parameters, Harmonization-parameters and Gradation-parameters, and/or
- a X-ray parameter which has an influence over the dose or image quality is preferably introduced into the training-unit with the premise of repeating all steps in the training and automation-unit, and/or
- the actual DI has been calculated and stored by the medical imaging system for each X-ray sample dataset or in turn the actual DI can be derived from data provided from the medical imaging system when an acquisition has been performed.

5. Method as claimed by one of the preceding claims, wherein the X-ray sample datasets (D) are preprocessed according to the principles of data preparation for machine learning, wherein the extracted data is preferably homogenized and/or arranged in a feature-vector.

6. Method as claimed by one of the preceding claims, wherein at least two different algorithms for machine learning are used during a test-training phase, wherein the results of the algorithms are compared with each other based on machine learning principles with respect to the performance and complexity metrics and the algorithm with the best metric results is chosen for further training of the automation-unit (6) .

7. Automation-unit (6) for automating X-ray acquisition and visualization at a minimum dose manufactured by a method as claimed by one of claims 1 to 6.

8. Automation-method for automation of X-ray image acquisition and visualization at a minimum dose, comprising the steps:

- providing an automation-unit (6) as claimed in claim 7,
- providing medical task parameters ($P_M$) about a planned image-acquisition,

- calculating, with the automation-unit (6), optimal values for acquisition parameters ($P_A$) and/or processing parameters ($P_P$) based on the medical task parameters ($P_M$),
- providing the calculated optimal values for acquisition parameters ($P_A$) and/or of processing parameters ($P_P$) to be used for image acquisition.

9. Automation-method as claimed by claim 8, wherein a value for an acquisition parameter ($P_A$) and/or a processing parameter ($P_P$) is manually adjusted by a user, followed by the steps:

- calculation of a calculated Deviation Index $DI_C$ by the automation unit (6), preferably by extracting values of acquisition parameters ($P_A$) and/or of processing parameters ($P_P$) from the medical task parameters ($P_M$) and calculating the calculated Deviation Index $DI_C$ based on the extracted values of acquisition parameters ($P_A$) and/or of processing parameters ($P_P$),
- comparing the calculated Deviation Index ($DI_C$) with an acceptable range for the Deviation Index ($DI_R$),
- provide a correction or a warning if the calculated Deviation Index $DI_C$ exceeds the acceptable range.

10. Automation-method as claimed by claim 8 or 9, wherein an image acquisition is automatically started with the adjusted and/or the calculated optimal values of acquisition parameters ($P_A$) and/or processing parameters ($P_P$) if the patient is positioned correctly.

11. Automation-method as claimed by one of claims 8 to 10, comprising the steps:

- providing an interface to the automation-unit (6),
- providing the calculated optimal values of acquisition parameters ($P_A$) and/or processing parameters ($P_P$) preferably stored in a data base in a service cloud as the results of the training-unit (7) for different medical tasks and to be further used for the automatic image acquisition and visualization,
- providing a scheduler to update the data provided to the automation-unit (6) at the customer site.

12. Automation device (10) for automating X-ray acquisition and visualization at a minimum dose, comprising

- a data-interface (12) to receive data of an X-ray system,
- a dataset-unit (16) with datasets comprising headers of medical image files, comprising information about

a) the Deviation Index (DI) or data from which the Deviation Index (DI) can be derived,
b) acquisition parameters and/or processing parameters, and
c) medical task parameters,

- an automation-unit (6) as claimed in claim 7,
- a data interface (13) to provide values for acquisition parameters and/or processing parameters for an X-ray system and/or to control an X-ray system.

13. Medical X-ray imaging system (1) comprising a number of X-ray imaging systems and an automation device (10) as claimed in claim 12.

14. A computer program product comprising a computer program that is directly loadable into a memory of a control unit of a computing unit and which comprises program elements for performing steps of the method according to claim 1 to 6 and/or the automation method according to claims 8 to 11 when the computer program is executed in the computing unit.

15. A computer-readable medium on which is stored program elements that can be read and executed by a computer unit in order to perform steps of the method according to claim 1 to 6 and/or the automation method according to claims 8 to 11 when the program elements are executed by the computer unit.

**Patentansprüche**

1. Verfahren zur Erstellung einer Automatisierungseinheit (6) zur Automatisierung der Röntgenbilderfassung und -visualisierung bei minimaler Dosis umfassend die folgenden Schritte:

- das Bereitstellen einer computerisierten Trainingseinheit (7), wobei die Trainingseinheit dazu entworfen ist, eine Automatisierungseinheit (6) mit einem maschinellen Lernverfahren zu trainieren,
- das Bereitstellen einer Automatisierungseinheit (6) bei der Trainingseinheit (7),
- das Bereitstellen von Röntgenprobendatensätzen (D) umfassend Auskunft über

a) den Abweichungsindex (DI) oder Daten von welchen der Abweichungsindex (DI) abgeleitet werden kann,
b) Werte von Erfassungsparametern ($P_A$) und/oder von Verarbeitungsparametern ($P_P$) und
c) medizinische Aufgabenparameter, einer Röntgenbilddatei,

- das Entnehmen von Daten aus den Röntgendatensätzen (D), wobei die entnommenen Daten den Abweichungsindex (DI) und vordefinierte Werte von Erfassungsparametern ($P_A$) und/oder von Verarbeitungsparametern ($P_P$) aus den Röntgenprobendatensätzen umfassen,
- das Bilden einer Anzahl von Datengruppen (G) aus den entnommenen Daten, wobei für das Trainieren der Automatisierungseinheit (6) Datensätze (D), welche im Wesentlichen identische medizinische Aufgabenparameter betreffen, in einer Datengruppe (G) gruppiert werden;
- das Trainieren der Automatisierungseinheit (6) mit der Trainingseinheit (7) basierend auf den Prinzipien des maschinellen Lernens unter Verwendung der Anzahl von Datengruppen (G);

wobei der Abweichungsindex (DI) die Veränderung des tatsächlichen Expositionsindexes (EI) im Vergleich zu einem idealen Ziel-Expositionsindex (TEI) für einen Patienten misst.

2. Verfahren nach Anspruch 1, wobei ein Trainingsmodell durch die folgenden Schritte erstellt wird:

- das Kombinieren von Werten von verarbeiteten vordefinierten Erfassungsparametern ($P_A$) und/oder Verarbeitungsparametern ($P_P$) eines Röntgenprobendatensatzes (D), welche mit Gewichtskoeffizienten ($W_1$, $W_2$) gewichtet werden,
- das Durchführen eines Trainingsschrittes mit den kombinierten Werten in Bezug auf die tatsächliche Abweichungseinheit (DI) des Röntgenprobendatensatzes (D) oder einem vergleichbaren Einheitswert des Röntgenprobendatensatzes (D), welcher die gleiche/eine ähnliche Definition aufweist,
- das Wiederholen dieser Schritte mit unterschiedlichen Röntgenprobendatensätzen (D).

3. Verfahren nach einem der vorhergehenden Ansprüche, wobei in einer Datengruppe (G) die gleiche Kombination von Werten von Erfassungsparametern ($P_A$) und/oder Verarbeitungsparametern ($P_P$) gegenüber der Datensätze dieser Datengruppe für das Trainieren angewendet wird, wobei vorzugsweise die Gewichtskoeffizienten ($W_1$, $W_2$) durch das Lernen des Trainierens der Automatisierungseinheit (6) berechnet werden.

4. Verfahren nach einem der vorhergehenden Ansprüche, wobei aus den Röntgenprobendatensätzen (D) die Eingabedaten für die Trainingseinheit (7) und die Auskunft, um Datengruppen (G) zu bilden, entnommen werden, wobei:

- die Erfassungsparameter Parameter umfassen, welche während einer Erfassung einer Röntgenbildprobe verwendet werden, vorzugsweise Parameter ausgewählt aus der Gruppe umfassend Kollimationsparameter, mAs-Parameters, Expositionskorrekturparameter und Filterparameter, und/oder
- die Verarbeitungsparameter Parameter umfassen, welche dazu verwendet werden können, eine Bildprobe zu verarbeiten, vorzugsweise Parameter ausgewählt aus der Gruppe umfassend Verstärkungsparameter, Fensterungsparameter, Harmonisierungsparameter und Abstufungsparameter, und/oder
- ein Röntgenparameter, welcher Einfluss auf die Dosis oder Bildqualität hat, vorzugsweise in die Trainingseinheit eingebracht wird, mit der Voraussetzung des Wiederholens der gesamten Schritte in der Trainings- und Automatisierungseinheit, und/oder
- der tatsächliche DI vom medizinischen Abbildungssystem für jeden Röntgenprobendatensatz berechnet und gespeichert worden ist oder wiederum der tatsächlich DI aus Daten, welche vom medizinischen Abbildungssystem bereitgestellt werden, wenn eine Erfassung durchgeführt worden ist, abgeleitet werden kann.

5. Verfahren nach einem der vorhergehenden Ansprüche, wobei die Röntgenprobendatensätze (D) gemäß den Prinzipien der Datenvorbereitung für das maschinelle Lernen vorverarbeitet werden, wobei die entnommenen Daten vorzugsweise homogenisiert und/oder in einem Merkmalsvektor angeordnet werden.

6. Verfahren nach einem der vorhergehenden Ansprüche, wobei mindestens zwei unterschiedliche Algorithmen für das maschinelle Lernen während einer Test-Trainingsphase verwendet werden, wobei die Ergebnisse der Algorithmen basierend auf maschinellen Lernprinzipien in Bezug auf die Leistungs- und Komplexitätsmetriken miteinander verglichen werden und der Algorithmus mit den besten Metrikergebnissen für das zusätzliche Trainieren der Automatisierungseinheit (6) gewählt wird.

7. Automatisierungseinheit (6) zur Automatisierung der Röntgenerfassung und -visualisierung bei minimaler Dosis hergestellt durch ein Verfahren nach einem der Ansprüche 1 bis 6.

8. Automatisierungsverfahren zur Automatisierung der Röntgenbilderfassung und -visualisierung bei minimaler Dosis, umfassend die folgenden Schritte:

   - das Bereitstellen einer Automatisierungseinheit (6) nach Anspruch 7,
   - das Bereitstellen von medizinischen Aufgabenparametern ($P_M$) über eine geplante Bilderfassung,
   - das Berechnen, mit der Automatisierungseinheit (6), von optimalen Werten für Erfassungsparameter ($P_A$) und/oder Verarbeitungsparameter ($P_P$) basierend auf den medizinischen Aufgabenparametern ($P_M$),
   - das Bereitstellen der berechneten optimalen Werte für Erfassungsparameter ($P_A$) und/oder von Verarbeitungsparametern ($P_P$), welche für die Bilderfassung zu verwenden sind.

9. Automatisierungsverfahren nach Anspruch 8, wobei ein Wert für einen Erfassungsparameter ($P_A$) und/oder einen Verarbeitungsparameter ($P_P$) vonseiten eines Benutzers manuell eingestellt wird, gefolgt von den folgenden Schritten:

   - das Berechnen eines berechneten Abweichungsindexes Die vonseiten der Automatisierungseinheit (6), vorzugsweise indem Werte von Erfassungsparametern ($P_A$) und/oder von Verarbeitungsparametern ($P_P$) aus den medizinischen Aufgabenparametern ($P_M$) entnommen werden und der berechnete Abweichungsindex Die basierend auf den entnommenen Werten von Erfassungsparametern ($P_A$) und/oder von Verarbeitungsparametern ($P_P$) berechnet wird,
   - das Vergleichen des berechneten Abweichungsindexes ($DI_C$) mit einem akzeptablen Bereich für den Abweichungsindex ($DI_R$),
   - das Bereitstellen einer Korrektur oder einer Warnung, wenn der berechnete Abweichungsindex Die den akzeptablen Bereich überschreitet.

10. Automatisierungsverfahren nach Anspruch 8 oder 9, wobei eine Bilderfassung mit den eingestellten und/oder den berechneten optimalen Werten von Erfassungsparametern ($P_A$) und/oder Verarbeitungsparametern ($P_P$) automatisch gestartet wird, wenn der Patient richtig positioniert ist.

11. Automatisierungsverfahren nach einem der Ansprüche 8 bis 10, umfassend die folgenden Schritte:

   - das Bereitstellen einer Schnittstelle zur Automatisierungseinheit (6),
   - das Bereitstellen der berechneten optimalen Werten von Erfassungsparametern ($P_A$) und/oder Verarbeitungsparametern ($P_P$), welche vorzugsweise in einer Datenbank in einer Service-Cloud gespeichert sind, als Ergebnisse der Trainingseinheit (7) für unterschiedliche medizinische Aufgaben und welche für die automatische Bilderfassung und -visualisierung zusätzlich zu verwenden sind,
   - das Bereitstellen eines Planers, um die Daten, welche der Automatisierungseinheit (6) bereitgestellt werden, am Kundenstandort zu aktualisieren.

12. Automatisierungsvorrichtung (10) zur Automatisierung der Röntgenerfassung und -visualisierung bei minimaler Dosis, umfassend

   - eine Datenschnittstelle (12), um Daten eines Röntgensystems zu empfangen,
   - eine Datensatzeinheit (16) mit Datensätzen, welche Köpfe von medizinischen Bilddateien umfassen, umfassend Auskunft über

      a) den Abweichungsindex (DI) oder Daten von welchen der Abweichungsindex (DI) abgeleitet werden kann,
      b) Erfassungsparameter und/oder Verarbeitungsparameter, und
      c) medizinische Aufgabenparameter,

   - eine Automatisierungseinheit (6) nach Anspruch 7,

- eine Datenschnittstelle (13), um Werte für Erfassungsparameter und/oder Verarbeitungsparameter für ein Röntgensystem bereitzustellen und/oder um ein Röntgensystem zu steuern.

13. Medizinisches Röntgenabbildungssystem (1) umfassend eine Anzahl von Röntgenabbildungssystemen und einer Automatisierungsvorrichtung (10) nach Anspruch 12.

14. Computerprogrammprodukt umfassend ein Computerprogramm, welches direkt in einem Speicher einer Steuereinheit einer Recheneinheit geladen werden kann und welches Programmelemente zum Durchführen von Schritten des Verfahrens nach Anspruch 1 bis 6 und/oder des Automatisierungsverfahren nach den Ansprüchen 8 bis 11 umfasst, wenn das Computerprogramm in der Recheneinheit ausgeführt wird.

15. Computerlesbares Medium, auf welchem Programmelemente gespeichert sind, welche vonseiten einer Computereinheit gelesen und ausgeführt werden können, um Schritte des Verfahrens nach Anspruch 1 bis 6 und/oder des Automatisierungsverfahren nach den Ansprüchen 8 bis 11 durchzuführen, wenn die Programmelemente vonseiten der Computereinheit ausgeführt werden.

## Revendications

1. Procédé pour la création d'une unité d'automatisation (6) pour l'automatisation d'acquisition et de visualisation d'images à rayons X à une dose minimale comprenant les étapes suivantes :

   - fournir une unité d'entraînement informatisée (7), dans lequel l'unité d'entraînement est conçue pour entraîner une unité d'automatisation (6) avec un procédé d'apprentissage automatique,
   - fournir une unité d'automatisation (6) à l'unité d'entraînement (7),
   - fournir des ensembles de données d'échantillons de rayons X (D) comprenant des informations sur

     a) l'indice de déviation (DI) ou des données à partir desquels l'indice de déviation (DI) peut être dérivé,
     b) des valeurs de paramètres d'acquisition ($P_A$) et/ou de paramètres de traitement ($P_P$) et
     c) des paramètres de tâche médicale,
     d'un fichier d'image à rayons X,

   - extraire des données à partir des ensembles de données de rayons X (D), dans lequel les données extraites comprennent l'indice de déviation (DI) et des valeurs prédéfinies de paramètres d'acquisition ($P_A$) et/ou de paramètres de traitement ($P_P$) à partir des ensembles de données d'échantillons de rayons X,
   - former plusieurs groupes de données (G) à partir des données extraites, dans lequel, pour l'entraînement de l'unité d'automatisation (6), des ensembles de données (D) concernant des paramètres de tâche médicale essentiellement identiques sont regroupés dans un groupe de données (G) ;
   - entraîner l'unité d'automatisation (6) avec l'unité d'entraînement (7) sur la base des principes d'apprentissage automatique en utilisant plusieurs groupes de données (G) ;

   dans lequel l'indice de déviation (DI) mesure la variation de l'indice d'exposition réel (EI) par rapport avec un indice d'exposition cible idéal (TEI) pour un patient.

2. Procédé selon la revendication 1, dans lequel un modèle d'entraînement est construit au moyen des étapes suivantes :

   - combiner des valeurs de paramètres d'acquisition prédéfinis traités ($P_A$) et/ou de paramètres de traitement ($P_P$) d'un ensemble de données d'échantillons de rayons X (D) qui sont pondérés avec des coefficients de pondération ($W_1$, $W_2$),
   - effectuer une étape d'entraînement avec les valeurs combinés par rapport à l'unité de déviation réelle (DI) de l'ensemble de données d'échantillons de rayons X (D) ou une valeur d'unité comparable de l'ensemble de données d'échantillons de rayons X (D) qui a une définition identique/similaire,
   - répéter ces étapes avec des ensembles de données d'échantillons de rayons X (D) différents.

3. Procédé selon l'une quelconque des revendications précédentes, dans lequel dans un groupe de données (G) la même combinaison de valeurs de paramètres d'acquisition ($P_A$) et/ou paramètres de traitement ($P_P$) sur les ensembles de données de ce groupe de données est appliquée pour l'entraînement, dans lequel préférablement les

coefficients de pondération ($W_1$, $W_2$) sont calculés par l'apprentissage de l'entraînement de l'unité d'automatisation (6).

4. Procédé selon l'une quelconque des revendications précédentes, dans lequel à partir des ensembles de données d'échantillons de rayons X (D), les données d'entrée pour l'unité d'entraînement (7) et les informations pour former des groupes de données (G) sont extraites,
dans lequel :

- les paramètres d'acquisition comprennent des paramètres utilisés lors d'une acquisition d'un échantillon d'image à rayons X, préférablement des paramètres choisis parmi le groupe comprenant des paramètres de collimation, des paramètres mAs, des paramètres et correction de l'exposition et des paramètres de filtre, et/ou
- les paramètres de traitement comprennent des paramètres qui peuvent être utilisés pour le traitement d'un échantillon d'image, préférablement des paramètres choisis parmi le groupe comprenant des paramètres d'amplification, des paramètres de fenêtrage, des paramètres d'harmonisation et des paramètres de gradation, et/ou
- un paramètre de rayons X qui a une influence sur la dose ou la qualité d'image est préférablement introduit dans l'unité d'entraînement avec la prémisse de répéter toutes les étapes dans l'unité d'entraînement et d'automatisation, et/ou
- le DI réel a été calculé et stocké par le système d'imagerie médical pour chaque ensemble de données d'échantillons de rayons X ou à son tour le DI réel peut être dérivé à partir de données fournies à partir du système d'imagerie médical lorsqu'une acquisition a été effectuée.

5. Procédé selon l'une quelconque des revendications précédentes, dans lequel les ensembles de données d'échantillons de rayons X (D) sont prétraités selon les principes de préparation de données pour l'apprentissage automatique, dans lequel les données extraites sont préférablement homogénéisées et/ou disposées dans un vecteur de caractéristiques.

6. Procédé selon l'une quelconque des revendications précédentes, dans lequel au moins deux algorithmes différents pour l'apprentissage automatique sont utilisés pendant une phase d'apprentissage de test, dans lequel les résultats des algorithmes sont comparés entre eux sur la base des principes d'apprentissage automatique par rapport à des métriques de performance et de complexité et l'algorithme avec les meilleurs résultats de métrique est choisi pour l'entraînement additionnel de l'unité d'automatisation (6).

7. Unité d'automatisation (6) pour l'automatisation d'acquisition et de visualisation de rayons X à une dose minimale fabriquée au moyen d'un procédé selon l'une des revendications 1 à 6.

8. Procédé d'automatisation pour l'automatisation d'acquisition et de visualisation d'images à rayons X à une dose minimale, comprenant les étapes suivantes :

- fournir une unité d'automatisation (6) selon la revendication 7,
- fournir des paramètres de tâche médicale ($P_M$) sur une acquisition d'image planifiée,
- calculer, avec l'unité d'automatisation (6), des valeurs optimales pour des paramètres d'acquisition ($P_A$) et/ou des paramètres de traitement ($P_P$) sur la base des paramètres de tâche médicale ($P_M$),
- fournir les valeurs optimales calculées pour les paramètres d'acquisition ($P_A$) et/ou les paramètres de traitement ($P_P$) à utiliser pour l'acquisition d'images.

9. Procédé d'automatisation selon la revendication 8, dans lequel une valeur pour un paramètre d'acquisition ($P_A$) et/ou un paramètre de traitement ($P_P$) est manuellement ajustée par un utilisateur, suivi par les étapes suivantes :

- calculer un indice de déviation DIc par l'unité d'automatisation (6), préférablement en extrayant des valeurs de paramètres d'acquisition ($P_A$) et/ou de paramètres de traitement ($P_P$) à partir des paramètres de tâche médicale ($P_M$) et en calculant l'indice de déviation calculé DIc sur la base des valeurs extraites de paramètres d'acquisition ($P_A$) et/ou de paramètres de traitement (Pp),
- comparer l'indice de déviation calculé (DIc) avec une plage acceptable pour l'indice de déviation ($DI_R$),
- fournir une correction ou une alerte si l'indice de déviation calculé DIc dépasse la plage acceptable.

10. Procédé d'automatisation selon les revendications 8 ou 9, dans lequel une acquisition d'image est lancée automatiquement avec les valeurs optimales ajustées et/ou calculées de paramètres d'acquisition ($P_A$) et/ou paramètres de traitement ($P_P$) si le patient est correctement positionné.

**11.** Procédé d'automatisation selon l'une des revendications 8 à 10, comprenant les étapes suivantes :

- fournir une interface avec l'unité d'automatisation (6),
- fournir les valeurs optimales calculées de paramètres d'acquisition ($P_A$) et/ou paramètres de traitement ($P_P$) préférablement stockées dans une base de données dans un nuage de service en tant que les résultats de l'unité d'entraînement (7) pour différentes tâches médicales et pour être utilisées additionnellement pour l'acquisition et visualisation d'images automatiques,
- fournir un planificateur pour mettre à jour les données fournies à l'unité d'automatisation (6) dans le site du client.

**12.** Dispositif d'automatisation (10) pour l'automatisation d'acquisition et de visualisation de rayons X à une dose minimale, comprenant

- une interface de données (12) pour recevoir des données d'un système de rayons X,
- une unité d'ensemble de données (16) avec des ensembles de données comprenant des entêtes de fichiers d'images médicales, comprenant des informations sur

a) l'indice de déviation (DI) ou des données à partir desquels l'indice de déviation (DI) peut être dérivé,
b) des paramètres d'acquisition et/ou des paramètres de traitement, et
c) des paramètres de tâche médicale,

- une unité d'automatisation (6) selon la revendication 7,
- une interface de données (13) pour fournir des valeurs pour des paramètres d'acquisition et/ou paramètres de traitement pour un système de rayons X et/ou pour commander un système de rayons X.

**13.** Système d'imagerie de rayons X médical (1) comprenant plusieurs systèmes d'imagerie de rayons X et un dispositif d'automatisation (10) selon la revendication 12.

**14.** Produit de programme informatique comprenant un programme informatique qui peut être chargé directement dans une mémoire d'une unité de commande d'une unité informatique et qui comprend des éléments de programme pour effectuer des étapes du procédé selon les revendications 1 à 6 et/ou le procédé d'automatisation selon les revendications 8 à 11 lorsque le programme informatique est exécuté dans l'unité informatique.

**15.** Support lisible par ordinateur sur lequel sont stockés des éléments de programme qui peuvent être lus et exécutés par une unité informatique afin d'effectuer des étapes du procédé selon les revendications 1 à 6 et/ou le procédé d'automatisation selon les revendications 8 à 11 lorsque les éléments de programme sont exécutés par l'unité informatique.

FIG 1

FIG 2

FIG 3

# FIG 4

# FIG 5

DICOM-HEADER INFORMATION EXTRACTOR

DEVIATION INDEX

X-ray Parameters

ACQUISITION parameter extractor

IMAGE PROCESSING parameter extractor

DATA PREPARATION Unit

DATA UNIT CONVERSION / HOMOGENEITY

CLINICAL APPLICATION Determination Module

Feature Vectors Preparation / Training Data Input

Machine Learning Unit

# FIG 6

Training Data Set

Machine learning algorithms

BUILD the MODEL

TRAIN the MODEL

OPTIMIZATION GOAL
DI=0

MODEL VALIDATION Unit

Performance Metrics

Model Choice

OPTIMAL X-Ray Parameters
CALCULATION

OPTIMAL ACQUISITION
parameters

OPTIMAL IMAGE
PROCESSING parameters

OPTIMAL
PARAMETERS

X-Ray Controlling Unit

FIG 7

Patient

DATA PROCESSOR
UNIT

OPTIMAL
PARAMETERS

VISUALIZATION X-Ray
OPTIMAL PATIENT
PARAMETERS UNIT

Correction/
Warning
Manager

No — Variation of a
PARAMETER?

Yes

PREDICTION of DI based
on TRAINING MODEL

No — DI clinically
acceptable?

Yes

Acquisition & Image Visualization

Update
Scheduler

CLOUD
SERVICES

Feedback Loop
Controller

# FIG 8

| Actual Exposure Index | Target Exposure Index | Deviation Index (DI) | DI % Target |
|---|---|---|---|
| 1300 | 500 | 4 | 160 |
| 1000 | 500 | 3 | 100 |
| 800 | 500 | 2 | 60 |
| 630 | 500 | 1 | 26 |
| 500 | 500 | 0 | 0 |
| 400 | 500 | -1 | -20 |
| 300 | 500 | -2 | -40 |
| 250 | 500 | -3 | -50 |
| 200 | 500 | -4 | -60 |

| DI | Range Action |
|---|---|
| > +3.0 | Excessive patient radiation exposure Repeat only if relevant anatomy is clipped or "burned out" Require immediate management followup. |
| +1 to +3.0 | Overexposure: Repeat only if relevant anatomy is clipped or "burned out" |
| -0.5 to +0.5 | Target range |
| Less than -1.0 | Underexposed: Consult radiologist for repeat |
| Less than -3.0 | Repeat |

**EP 3 646 791 B1**

**REFERENCES CITED IN THE DESCRIPTION**

**Patent documents cited in the description**

- JP 2017007249 A